Europäisches Patentamt

European Patent Office

Office européen des brevets

(11) Veröffentlichungsnummer: **0 459 401 A1**

## (12) EUROPÄISCHE PATENTANMELDUNG

(21) Anmeldenummer: **91108701.3**

(22) Anmeldetag: **28.05.91**

(51) Int. Cl.⁵: **H01F 5/00, A61N 2/00**

(30) Priorität: **29.05.90 DE 9006056 U**

(43) Veröffentlichungstag der Anmeldung:
**04.12.91 Patentblatt 91/49**

(84) Benannte Vertragsstaaten:
**AT CH DE ES FR GB IT LI SE**

(71) Anmelder: **Kraus, Werner, Dipl.-Ing.
Augustenstrasse 41
W-8000 München 2(DE)**

(72) Erfinder: **Kraus, Werner, Dipl.-Ing.
Augustenstrasse 41
W-8000 München 2(DE)**

(74) Vertreter: **von Bezold, Dieter, Dr. et al
Dr. Dieter von Bezold Dipl.-Ing. Peter Schütz
Dipl.-Ing. Wolfgang Heusler Brienner Strasse
52
W-8000 München 2(DE)**

(54) **Applikatorspule für die Magnetfeldtherapie.**

(57) Applikatorspule für ein elektromagnetisches Therapiegerät, die im wesentlichen die Form einer oval verformten Kugelzone hat und sich vor allem dazu eignet, niederfrequente magnetische Wechselfelder auf vorspringende Teile des menschlichen oder tierischen Körpers zur Einwirkung zu bringen, wie den Schulterbereich oder das Gesicht des Menschen.

FIG. 1

EP 0 459 401 A1

Die vorliegende Erfindung betrifft eine Applikatorspule für die Magnetfeldtherapie.

Aus dem Dokument DE-A-24 32 493 ist eine Applikatorspule in Form einer mehrlagigen Zylinder- oder Solenoidspule mit einem ovalen oder ellipsenförmigen Querschnitt bekannt.

Die Applikatorspulen der hier interessierenden Art dienen in der Human- und Tiermedizin zur Heilbehandlung mittels niederfrequenter, möglichst oberwellenarmer magnetischer Wechselfelder, deren Frequenz vorzugsweise unter 20 Hz liegt. Das Magnetfeld kann unmittelbar zur Behandlung dienen oder zur Induktion von niederfrequenten Wechselströmen in einer implantierten Aufnehmerspule verwendet werden, die einen Teil einer Vorrichtung zur Förderung des Knochen- bzw. Gewebewachstums bildet (siehe z.B. die Dokumente DE-C-19 18 299 und DE-A-21 16 869)

Die bekannten hohlzylinderförmigen Applikatorspulen dienen in erster Linie zur Behandlung von Extremitäten oder des Rumpfes und werden hierfür auf den zu behandelnden Körperteil aufgeschoben. Sie eignen sich jedoch weniger zur gezielten Behandlung vorspringender Körperteile, wie einer Schulter und des Gesichtsbereiches.

Der vorliegenden Erfindung liegt dementsprechend die Aufgabe zugrunde, eine Applikatorspule zum Erzeugen eines niederfrequenten Magnetfeldes für ein elektromagnetisches Therapiegerät anzugeben, welches sich besonders für die gezielte Behandlung vorstehender Teile des menschlichen und gegebenenfalls tierischen Körpers eignet.

Diese Aufgabe wird durch eine Applikatorspule gelöst, die im wesentlichen die Form einer oval verformten Kugelzone hat.

Weiterbildungen und vorteilhafte Ausgestaltungen der erfindungsgemäßen Applikatorspule sind Gegenstand der Unteransprüche.

Dadurch, daß die Applikatorspule im wesentlichen die Form einer oval oder nach Art eines Trapezes mit abgerundeten Ecken verformten Kugelzone hat, also kalottenartig mit einem Querschnitt, der sich von einer größeren Öffnung zu einer kleineren Öffnung hin verjüngt, ausgebildet ist, läßt sie sich eng an vorstehende Körperteile, wie das Gesicht oder eine Schulter anlegen, wodurch eine effektive Behandlung gewährleistet ist.

Bei bevorzugten Weiterbildungen der vorliegenden Applikatorspule ist die Innenseite mit Düsen versehen, durch die ein Behandlungsfluid, wie ein Flüssigkeitsspray oder ein Gas auf die Oberfläche des behandelten Körperteils zur Einwirkung gebracht werden kann.

Im folgenden wird ein bevorzugtes Ausführungsbeispiel der Erfindung unter Bezugnahme auf die Zeichnungen näher erläutert. Es zeigen:

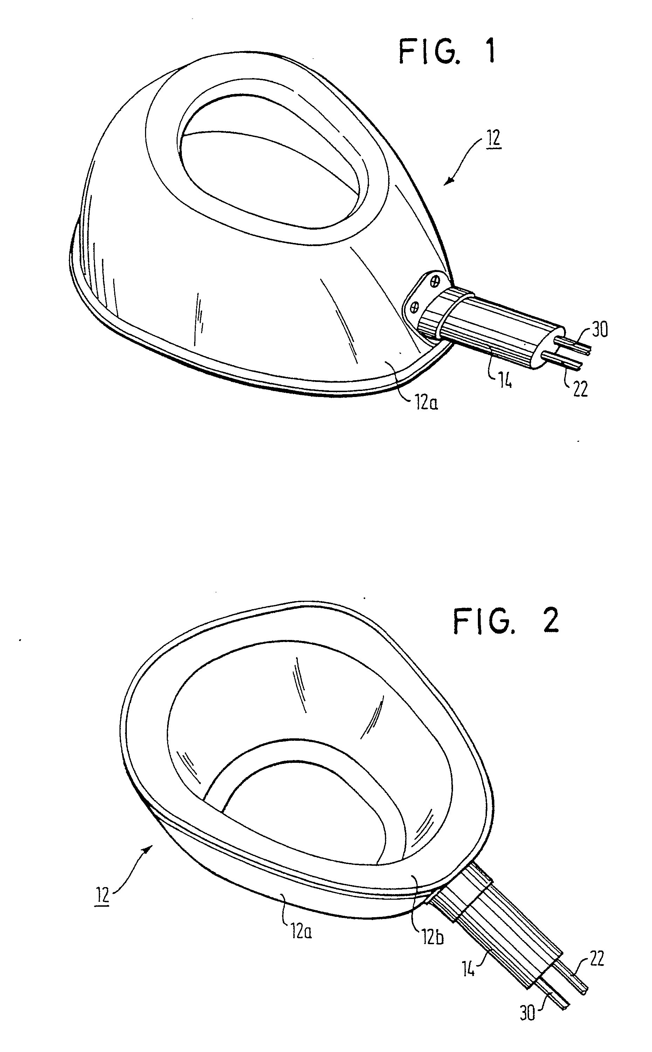

Figur 1    eine perspektivische Ansicht der Außenseite einer Applikatorspule gemäß einer bevorzugten Ausführungsform der Erfindung;

Figur 2    eine perspektivische Ansicht der Innenseite der Applikatorspule gemäß Figur 1 und

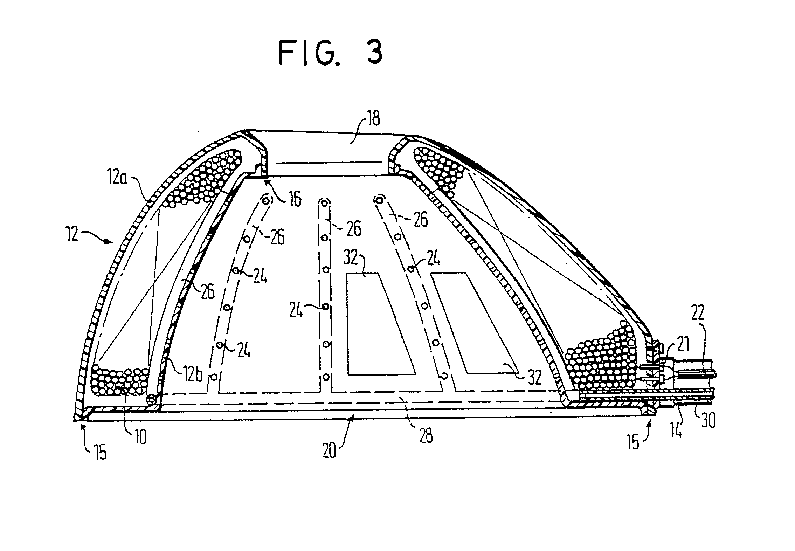

Figur 3    ein etwas vereinfachter Längsschnitt der Applikatorspule gemäß Figur 1 und 2.

Die in den Zeichnungen dargestellte Applikatorspule hat näherungsweise die Form einer eiförmig oder oval verformten Kugelzone. Sie enthält eine entsprechend geformte mehrlagige Drahtwicklung 10, deren Windungen im wesentlichen eiförmig sind oder die Form von Trapezen mit abgerundeten Ecken haben. Die Wicklungsdichte kann am weiteren Ende etwas größer sein als am engeren Ende der Spule. Die Wicklung 10 ist mit einem Kunststoffmantel 12 umgeben, der aus zwei Schalen 12a, 12b besteht, die bei 15, 16 flanschartige Ränder aufweisen, die miteinander verklebt oder verschweißt sind. Am schmäleren oder spitzeren Ende der äußeren Schale 12a ist ein Griff 14 angebracht. Die Applikatorspule hat eine engere Öffnung 18, die durch einen abgerundeten Teil der äußeren Schale 12a begrenzt wird. Am weiteren Ende 20 ist die Applikatorspule bis auf die Flansche 15 im wesentlichen eben. Die Spulenwicklung 10 ist mit Anschlüssen 21 versehen, die im Griff 14 untergebracht und mit einem Anschlußkabel 22 verbunden sind, mit dem die Applikatorspule an eine Generatoreinheit zur Erzeugung eines niederfrequenten, sinusförmigen Wechselstromes anschließbar ist, dessen Frequenz vorzugsweise zwischen etwa 2 und 20 Hz veränderbar ist.

Gemäß einer bevorzugten Ausgestaltung ist die Applikatorspule mit einer Anordnung zum Einspeisen eines Behandlungsfluids versehen. Sie weist hierfür an der durch die innere Schale 12b gebildeten Innenwand Öffnungen oder Düsen 24 auf, die über Stichleitungen 26 mit einer Speiseleitung 28 verbunden sind, an die wiederum ein Schlauch 30 angeschlossen ist. Der Schlauch 30 wird bei Verwendung der Applikatorspule mit einer Quelle für ein flüssiges oder gasförmiges Behandlungsmittel verbunden und gestattet es, die Magnetfeldbehandlung durch eine Behandlung der Oberfläche des behandelten Körperteils mit einem geeigneten Fluid, wie einem Heilmittel oder einem Kosmetikum, zu unterstützen.

An der Innenseite des Kunststoffmantels 12 können Elektroden 32 vorgesehen sein, mit denen ein elektrisches Feld galvanisch oder kapazitiv auf den zu behandelnden Körperteil zur Einwirkung gebracht werden kann. Diese Elektroden können über eine eigene Zuleitung (nicht dargestellt) mit einem geeigneten Generator für eine bezüglich null symmetrische oder unsymmetrische Wechselspannung oder eine unipolare Spannung mit niederfrequenter Welligkeit, also eine Gleichspannung, der eine Wechselspannung überlagert ist, verbindbar sein oder an eine eigene Aufnehmerspule (nicht dargestellt) angeschlossen sein, in der durch die Wicklung 10 eine entsprechende niederfrequente Spannung induzierbar ist. Diese Aufnehmerspule kann im Kunststoffmantel 12 drehbar angeordnet sein, um die Intensität und/oder Phasenlage der induzierten Wechselspannung einstellen zu können, und gegebenenfalls mit einer Gleichrichterschaltung gekoppelt sein, um eine bezüglich null unsymmetrische Wechselspannung oder eine wellige unipolare Spannung für die Elektroden zu erzeugen.

**Patentansprüche**

1. Applikatorspule für ein elektromagnetisches Therapiegerät, **dadurch gekennzeichnet,** daß sie im wesentlichen die Form einer oval verformten Kugelzone hat.

2. Applikatorspule nach Anspruch 1, **dadurch gekennzeichnet,** daß die Spulenwindungen eiförmig sind oder die Form von Trapezen mit abgerundeten Ecken haben.

3. Applikatorspule nach Anspruch 1 oder 2, **dadurch gekennzeichnet,** daß die Windungsdichte der Wicklung am weiteren Ende der Spule größer ist als am engeren Ende.

4. Applikatorspule nach Anspruch 1, 2 oder 3, **dadurch gekennzeichnet,** daß die Wicklung mit einem Kunststoffmantel (12) umgeben ist, der aus zwei miteinander verbundenen Schalen (12a, 12b) besteht.

5. Applikatorspule nach einem der vorhergehenden Ansprüche, **gekennzeichnet durch** eine Anordnung (24, 26, 28, 30) zum Einspeisen eines Fluids in das Innere der Spule.

6. Applikatorspule nach einem der vorhergehenden Ansprüche, **dadurch gekennzeichnet,** daß an der Innenseite Elektroden (32) zur Applikation eines elektrischen Feldes angeordnet sind.

7. Applikatorspule nach Anspruch 6, **dadurch gekennzeichnet,** daß die Elektroden mit einer eigenen Zuleitung für einen externen Spannungsgenerator versehen sind.

8. Applikatorspule nach Anspruch 6, **dadurch gekennzeichnet,** daß die Elektroden (32) mit einer Aufnehmerspule gekoppelt sind, in der durch die Wicklung der Applikatorspule ein Wechselstrom induzierbar ist.

9. Applikatorspule nach Anspruch 8, **dadurch gekennzeichnet,** daß die mit den Elektroden (32) gekoppelte Aufnehmerspule drehbar gelagert ist.

# FIG. 1

# FIG. 2

# FIG. 3

## EINSCHLÄGIGE DOKUMENTE

EP 91108701.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE - C2 - 2 116 869<br>(KRAUS)<br>* Fig. 1-8; Ansprüche 1-8 * | 1-7 | H 01 F 5/00<br>A 61 N 1/42 |
| D,A | DE - A - 1 918 299<br>(KRAUS)<br>* Fig. 1-4; Ansprüche 1-20 * | 1-7 | |
| D,A | DE - C2 - 2 432 493<br>(KRAUS)<br>* Fig. 1,2; Ansprüche 1-3 * | 1-7 | |
| A | EP - A2/A3 - 0 143 453<br>(SCHAUF)<br>* Zusammenfassung; Fig. 1,2 * | 1-7 | |
| A | GB - A - 2 217 990<br>(LIBER)<br>* Zusammenfassung; Fig. 1-8 * | 1-7 | |
| A | GB - A - 2 132 486<br>(ELECTRO-BIOLOGY INC.)<br>* Zusammenfassung; Fig. 1-8 * | 1-7 | **RECHERCHIERTE SACHGEBIETE (Int. Cl.5)** |
| A | GB - A - 283 236<br>(WILSHIRE)<br>* Gesamt * | 1-7 | H 01 F 5/00<br>A 61 N 1/00 |

Der vorliegende Recherchenbericht wurde für alle Patentansprüche erstellt.

| Recherchenort | Abschlußdatum der Recherche | Prüfer |
|---|---|---|
| WIEN | 04-09-1991 | VAKIL |